# EUROPÄISCHE PATENTSCHRIFT

(11) **EP 0 386 734 B1**
(45) Veröffentlichungstag und Bekanntmachung des Hinweises auf die Patenterteilung: **20.09.1995**
(21) Anmeldenummer: 90104353.9
(22) Anmeldetag: 07.03.1990
(51) Int. Cl.: C07K 4/02

(54) **Immunogene Regionen auf dem E-7-Protein des Humanen Papillomvirus Typ 16**
Immunogenic domains of the E-7 protein of the human papillome virus type 16
Domaines immunogènes de la protéine E-7 du virus du papillome humain de type 16

(30) Priorität: 10.03.1989 DE 3907721
(43) Veröffentlichungstag der Anmeldung: 12.09.1990
(73) Patentinhaber: BEHRINGWERKE Aktiengesellschaft, 35001 Marburg (DE)
(72) Erfinder: Bartsch, Dusan, Dr., D-6900 Heidelberg (DE); Gissmann, Lutz, Dr., Prof., D-6908 Wiesloch (DE); Müller, Martin, D-6900 Heidelberg (DE)

(56) Entgegenhaltungen:
- EP-A- 0 257 754
- EP-A- 0 375 555
- EP-A- 0 402 132
- THE EMBO JOURNAL, Band 6, Nr. 1, 1987, Seiten 139-144, IRL Press Ltd, Oxford, GB; K. SEEDORF et al.

## Beschreibung

Die Erfindung betrifft zwei immunogene Regionen auf dem Humanen Papillomvirus Typ 16 (HPV 16) E7-Protein, wobei die eine immunreaktive Region 3′ zu der Nukleotidposition 595 und die andere 3′ zu der Nukleotidposition 667 des HPV 16 Genoms lokalisiert ist.

Papillomviren lassen sich nicht in Kultur vermehren. Die Verwendung der HPV-DNA als Diagnostikum sowie die Gewinnung der Expressionsprodukte, deren Verwendung als Antigene, die Isolierung von Antikörpern und die Herstellung entsprechender Diagnostika setzt somit gentechnische Verfahren voraus.

Dürst et al., Proc. Natl. Acad. Sci. USA 80 (1983) 3813 - 3815 beschreiben einen neuen Typ von menschlichen Papillomviren (HPV), den sie als HPV 16 bezeichnen. Die DNA-Sequenz und die Genom-Organisation dieses Virus sind in Seedorf et al., Virology 145 (1985) 181 - 185 wiedergegeben.

Smotkin und Wettstein, Proc. Natl. Acad. Sci. 83, 4680 - 4684 (1986) identifizierten das E7-Transkript als das am häufigsten vorkommende HPV-Transkript in der CaSki Zellinie bzw. einem anderen Zervix-Karzinom, das HPV 16 DNA hauptsächlich als Plasmid enthält. Seedorf et al, EMBO J. 6, 139 - 144 (1987) zeigten dann, daß das E7-Protein das am häufigsten vorkommende virale HPV 16 Protein in solchen Zellinien ist, die HPV 16 in integrierter Form oder als Plasmid enthalten.

Das E7-Protein scheint darüberhinaus eine Rolle bei der Aufrechterhaltung des transformierten Phänotyps zu spielen. Weiterhin treten Antikörper gegen E7 Protein gehäuft bei Patienten mit Zervix-Karzinom auf. Der Nachweis von HPV 16 E7 Protein bzw. der dagegen gerichteten Antikörper ist deshalb von Interesse in der Diagnostik.

Bei der Analyse einer "shot gun" Expressionsbank klonierter HPV 16 DNA mit einem polyklonalen Antiserum gegen HPV16 E7 von Kaninchen wurden zwei immunreaktive Regionen innerhalb des E7-Proteins gefunden. Die erste Region liegt im N-terminalen Bereich von E7 und wird durch vier verschieden große Phagenklone repräsentiert:
I. 5′-ATG TTA GAT TTG CAA CCA GAG ACA ACT GAT CTC TAC TGT TAT GAG CAA-3′
II. 5′-ATG TTA GAT TTG CAA CCA GAG ACA ACT GAT CTC TAC
III. 5′-ATG TTA GAT TTG CAA CCA GAG ACA ACT
IV. 5′-ATG TTA GAT TTG CAA CCA GAG ACA
Dabei entspricht das 5′-Ende der Klone der Nukleotidposition 595 auf dem HPV 16 Genom
Entsprechend ist die Aminosäuresequenz der vier Klassen:
I. met leu asp leu gln pro glu thr thr asp leu tyr cys tyr glu gln
II. met leu asp leu gln pro glu thr thr asp leu tyr
III. met leu asp leu gln pro glu thr thr
IV. met leu asp leu gln pro glu thr
Die zweite immunreaktive Region von HPV 16 E7 wurde bei Nukleotidposition 667 auf dem HPV 16 Genom lokalisiert:
5′-GAT GAA ATA GAT GGT CCA GCT GGA CAA GCA GAA CCG GAC AGA GCC CAT TAC-3′
und enthält die 17 Aminosäuren:
asp glu ile asp gly pro ala gly gln ala glu pro asp arg ala his tyr
Die Erfindung betrifft folglich die o.g. beiden immunreaktiven Regionen, ihre Verwendung für Diagnostik, Therapie und als Arzneimittel bzw. Vakzine.

Die Erfindung ist ferner in den Beispielen und Patentansprüchen enthalten.

### Beispiele:

### 1. Herstellung der "shotgun"-Expressionsbank des HPV 16 Genoms.

Die im Bakterienplasmidvektor sp65 klonierte HPV 16 DNA wurde mit Ultraschallscherung und anschließender DNase I Behandlung auf eine durchschnittliche Fragmentgröße von etwa 100 Basenpaaren (bp) gebracht.

Die Enden dieser Fragmente wurden mit Hilfe von T4 DNA Polymerase und E.coli DNA Ligase aufgefüllt. Der Phagenexpressionsvektor fd-tet-J6 wurde mit PVU II geschnitten und die Fragmente 'blunt' einligiert. fd-tet-J6 ist von dem Bacteriophagen fd abgeleitet und von G. P. Smith, Science 228, 1315 - 1317 (1985) beschrieben worden.

### 2. Nachweis von immunogenen Reaktionen

Nach Ausplattieren der rekombinanten Phagen auf E.coli K91 wurden Replika auf Nitrocellulosefilter mit spezifischen Seren auf immunreaktive Phagen untersucht. 200 Rekombinanten reagierten mit dem Antiserum; 30 davon wurden mittels DNA-Sequenzanalyse weiter untersucht. Folgende Ergebnisse wurden erhalten:
1. Alle Rekombinanten enthalten E7-spezifische Sequenzen.
2. Innerhalb des E7-Proteins wurden zwei immunogene Regionen gefunden. Die erste Region wurde mittels 25 überlappender Klone identifiziert. Diese 25 Klone konnten in 4 Klassen eingeteilt werden:
   I. 5′-ATG TTA GAT TTG CAA CCA GAG ACA ACT GAT CTC TAC TGT TAT GAG CAA-3′
   II. 5′-ATG TTA GAT TTG CAA CCA GAG ACA ACT GAT CTC TAC
   III. 5′-ATG TTA GAT TTG CAA CCA GAG ACA ACT
   IV. 5′-ATG TTA GAT TTG CAA CCA GAG ACA

Dabei entspricht das 5′-Ende der Klone der Nukleotidposition 595 auf dem HPV 16 Genom.

Entsprechend ist die Aminosäuresequenz der vier Klassen:
I. met leu asp leu gln pro glu thr thr asp leu tyr cys tyr glu gln
II. met leu asp leu gln pro glu thr thr asp leu tyr
III. met leu asp leu gln pro glu thr thr
IV. met leu asp leu gln pro glu thr

Die minimale Größe dieser Region beträgt also 8 Aminosäuren. Da monoklonale Antikörper nur gegen Klasse I und II reagierten, das polyklonale Antiserum aber gegen alle 4 Klassen reagierte, sind mindestens 2 verschiedene Epitope auf dem Antigen der Klasse I bzw. II lokalisiert.

Entsprechend der Aminosäuresequenz der Klasse II wurde ein synthetisches Oligopeptid (met-leu-asp-leu-gln-pro-glu-thr-thr-asp-leu-tyr) im ELISA verwendet und zeigte deutliche Reaktion mit dem erwähnten polyklonalen Kaninchenserum gegen HPV 16 E7.

Die zweite immunreaktive Region von 17 Aminosäuren wurde in 5 Klonen aufgefunden und bei der Nukleotidposition 667 auf dem HPV 16 Genom lokalisiert:
5′-GAT GAA ATA GAT GGT CCA GCT GGA CAA GCA GAA CCG GAC AGA GCC CAT TAC-3′
entsprechend den 17 Aminosäuren
asp glu ile asp gly pro ala gly gln ala glu pro asp arg ala his tyr

## Patentansprüche (Patentansprüche für folgende(n) Vertragsstaat(en): AT, BE, CH, DE, DK, FR, GB, IT, LI, LU, NL, SE)

1. Immunogene Region von HPV16 E7-Protein, gekennzeichnet durch eine der Aminosäuresequenzen
I. met leu asp leu gln pro glu thr thr asp leu tyr cys tyr glu gln
II. met leu asp leu gln pro glu thr thr asp leu tyr
III. met leu asp leu gln pro glu thr thr
IV. met leu asp leu gln pro glu thr

2. Vakzine, die mindestens ein Peptid der immunogenen Regionen nach Anspruch 1 enthält.

3. Diagnostika zum Nachweis von spezifischen Antikörpern gegen HPV 16 E7-Proteine, gekennzeichnet durch einen Gehalt von mindestens einer der Aminosäuresequenzen nach Anspruch 1.

4. Poly- oder monoklonale Antikörper, die gegen mindestens eine immunogene Region nach Anspruch 1 gerichtet sind.

5. Diagnostikum, das poly- oder monoklonale Antikörper nach Anspruch 4 enthält.

## Patentansprüche (Patentansprüche für folgende(n) Vertragsstaat(en): ES)

1. Verfahren zur Herstellung einer Vakzine gegen HPV, dadurch gekennzeichnet, daß mindestens ein Peptid enthaltend eine der Aminosäuresequenzen
I. met leu asp leu gln pro glu thr thr asp leu tyr cys tyr glu gln
II. met leu asp leu gln pro glu thr thr asp leu tyr
III. met leu asp leu gln pro glu thr thr
IV. met leu asp leu gln pro glu thr
mit geeigneten Trägermaterialien versetzt werden.

2. Verfahren zur Herstellung von Diagnostika, dadurch gekennzeichnet, daß mindestens ein Peptid mit einer der in Anspruch 1 genannten Aminosäuresequenzen eingesetzt wird.

3. Verfahren zur Herstellung von poly- oder monoklonalen Antikörpern, dadurch gekennzeichnet, daß mindestens ein Peptid mit einer der in Anspruch 1 genannten Aminosäuresequenzen zur Immunisierung verwendet wird.

4. Verfahren zur Herstellung von Diagnostika, dadurch gekennzeichnet, daß nach Anspruch 3 hergestellte poly- oder monoklonale Antikörper verwendet werden.

## Claims (Claims for the following Contracting State(s): AT, BE, CH, DE, DK, FR, GB, IT, LI, LU, NL, SE)

1. An immunogenic region of HPV16 E7 protein, which has one of the amino acid sequences
I. met leu asp leu gln pro glu thr thr asp leu tyr cys tyr glu gln
II. met leu asp leu gin pro glu thr thr asp leu tyr
III. met leu asp leu gln pro glu thr thr
IV. met leu asp leu gln pro glu thr

2. A vaccine which contains at least one peptide from the immunogenic region as claimed in claim 1.

3. A diagnostic aid for detecting specific antibodies against HPV 16 E7 proteins, which contains at least one of the amino acid sequences as claimed in claim 1.

4. Poly- or monoclonal antibodies which are directed against at least one immunogenic region as claimed in claim 1.

5. A diagnostic aid which contains poly- or monoclonal antibodies as claimed in claim 4.

## Claims (Claims for the following Contracting State(s): ES)

1. A process for the preparation of a vaccine against HPV, which comprises mixing at least one peptide containing one of the amino acid sequences
I. met leu asp leu gln pro glu thr thr asp leu tyr cys tyr glu gln
II. met leu asp leu gin pro glu thr thr asp leu tyr
III. met leu asp leu gin pro glu thr thr
IV. met leu asp leu gln pro glu thr
with suitable carrier materials.

2. A process for the preparation of a diagnostic aid, which comprises using at least one peptide having one of the amino acid sequences mentioned in claim 1.

3. A process for the preparation of poly- or monoclonal antibodies, which comprises using at least one peptide having one of the amino acid sequences mentioned in claim 1 for the immunization.

4. A process for the preparation of a diagnostic aid, which comprises using poly- or monoclonal antibodies prepared as claimed in claim 3.

## Revendications (Revendications pour l'(les) Etat(s) contractant(s) suivant(s): AT, BE, CH, DE, DK, FR, GB, IT, LI, LU, NL, SE)

1. Domaine immunogène de la protéine E-7 d'HPV 16, caractérisé par l'une des séquences en acides aminés suivantes :
I. met leu asp leu gln pro glu thr thr asp leu tyr cys tyr glu gln
II. met leu asp leu gln pro glu thr thr asp leu tyr
III. met leu asp leu gln pro glu thr thr
IV. met leu asp leu gln pro glu thr

2. Vaccin comprenant au moins un peptide du domaine immunogène selon la revendication 1.

3. Produits de diagnostic pour la détection d'anticorps spécifiques dirigés contre la protéine E-7 d'HPV 16, caractérisés en ce qu'il renferment au moins une des séquences d'acides aminés selon la revendication 1.

4. Anticorps poly- ou monoclonaux dirigés contre au moins un domaine immunogène selon la revendication 1.

5. Produit de diagnostic renfermant un anticorps poly- ou monoclonal selon la revendication 4.

## Revendications (Revendications pour l'(les) Etat(s) contractant(s) suivant(s): ES)

1. Procédé de préparation d'un vaccin contre HPV, caractérisé en ce qu'on mélange au moins un peptide comportant une des séquences d'acides aminés suivantes :
I. met leu asp leu gln pro glu thr thr asp leu tyr cys tyr glu gln
II. met leu asp leu gln pro glu thr thr asp leu tyr
III. met leu asp leu gln pro glu thr thr
IV. met leu asp leu gln pro glu thr
avec des excipients appropriés.

2. Procédé de préparation de produits de diagnostic, caractérisé en ce qu'on met en oeuvre au moins un peptide comportant une des séquences d'acides aminés selon la revendication 1.

3. Procédé de préparation d'anticorps poly- ou monoclonaux, caractérisé en ce qu'on utilise pour l'immunisation au moins un peptide comportant une des séquences d'acides aminés selon la revendication 1.

4. Procédé de préparation de produits de diagnostic, caractérisés en ce qu'on utilise des anticorps poly- ou monoclonaux préparés selon le procédé de la revendication 3.
